# EUROPEAN PATENT APPLICATION

(11) **EP 3 553 749 A1**
(43) Date of publication of application: **16.10.2019**
(21) Application number: 18205511.1
(22) Date of filing: 09.11.2018
(51) Int. Cl.: G06T 11/00

(54) **APPARATUS AND METHOD FOR GENERATING TOMOGRAPHY IMAGE**

(30) Priority: 09.04.2018 KR 20180041245
(71) Applicant: Samsung Electronics Co., Ltd., Gyeonggi-do 16677 (KR)
(72) Inventor: Nam, Sang-nam, Suwon-si, Gyeonggi-do (KR); Lee, Kyoung-yong, Gyeonggi-do Hwaseong-si (KR)
(74) Representative: Gulde & Partner

(57) **Abstract**

A method of acquiring a tomography image includes acquiring a plurality of raw data by detecting an X-ray irradiated to an object from an X-ray generator a plurality of times, wherein an X-ray detector performs the detecting; setting at least a part of the object as a region of interest; and reconstructing the tomography image including the region of interest, based on the plurality of raw data, wherein the reconstructing of the tomography image includes: selecting filter kernels based on distances between positions of the X-ray generator that irradiated the X-ray to the region of interest and positions of voxels included in the region of interest; generating filter images by applying the selected filter kernels to the raw data corresponding to the positions of the X-ray generator that irradiated the X-ray; and reconstructing the tomography image from the filter images by back-projecting the filter images.

## Description

### CROSS-REFERENCE TO RELATED APPLICATION AND CLAIM OF PRIORITY

This application is based on and claims priority under 35 U.S.C. §119 to Korean Patent Application No. 10-2018-0041245, filed on April 9, 2018, in the Korean Intellectual Property Office, the disclosure of which is incorporated by reference herein in its entirety.

### BACKGROUND

### 1. Field

The disclosure relates to apparatuses and methods for generating tomography images.

### 2. Description of Related Art

A medical imaging apparatus is equipment for acquiring images of an internal structure of an object. A medical image processing apparatus is a non-invasive test apparatus that captures and processes images of structural details, internal tissues and fluid flow in the body and displays the same to a user. The user, such as a doctor, may diagnose a health condition and disease of a patient by using a medical image output from the medical image processing apparatus.

A representative example of an apparatus for capturing images of an object by irradiating a patient with X-rays is a computer tomography (CT) apparatus.

The CT apparatus, which is a tomography apparatus among medical image processing apparatuses, may provide a cross-sectional image of the object and express an internal structure (for example, an organ such as a kidney or a lung) of the object without overlapping the internal structure as compared with general X-ray apparatuses, and thus the CT apparatus may be widely used for accurate diagnosis of diseases. Hereinafter, a medical image acquired by the tomography apparatus is referred to as a tomography image.

In acquiring the tomography image, tomography imaging of the object may be performed using the tomography apparatus, and raw data may be acquired. The acquired raw data may be used to reconstruct the tomography image. In this regard, the raw data may be projection data acquired by projecting X-rays to the object or a sinogram that is a set of projection data.

A tomography apparatus according to the related art may generate a reconstructed image including non-uniform noise when reconstructing an image of the acquired raw data by using a filtered back-projection (FBP) method. As a result, when the user, such as the doctor, reads the image and diagnoses the disease, the accuracy of such reading and diagnosis may be degraded.

Therefore, when reconstructing an image, there is a need to generate a reconstructed image including uniform noise.

### SUMMARY

Provided are a tomography apparatus and method for generating a reconstructed image including uniform noise.

Additional aspects will be set forth in part in the description which follows and, in part, will be apparent from the description, or may be learned by practice of the presented embodiments.

In accordance with an aspect of the disclosure, a method of acquiring a tomography image includes acquiring a plurality of raw data by detecting, by an X-ray detector, an X-ray irradiated to an object from an X-ray generator a plurality of times; setting at least a part of the object as a region of interest; and reconstructing the tomography image including the region of interest, based on the plurality of raw data, wherein the reconstructing of the tomography image includes: selecting filter kernels based on distances between positions of the X-ray generator that irradiated the X-ray to the region of interest and positions of voxels included in the region of interest; generating filter images by applying the selected filter kernels to the raw data corresponding to the positions of the X-ray generator that irradiated the X-ray; and reconstructing the tomography image from the filter images by back-projecting the filter images.

In accordance with another aspect of the disclosure, an apparatus for acquiring a tomography image includes an X-ray generator configured to irradiate an X-ray to an object a plurality of times; an X-ray detector configured to acquire a plurality of raw data by detecting the irradiated X-ray; and a processor configured to set at least a part of the object as a region of interest and reconstruct the tomography image including the region of interest based on the plurality of raw data, wherein the processor is configured to select filter kernels based on distances between positions of the X-ray generator that irradiated the X-ray to the region of interest and positions of voxels included in the region of interest, generate filter images by applying the selected filter kernels to the raw data corresponding to the positions of the X-ray generator that irradiated the X-ray, and reconstruct the tomography image from the filter images by back-projecting the filter images.

In accordance with another aspect of the disclosure, a computer program product including a non-transitory computer readable storage medium, wherein the non-transitory computer readable storage medium includes instructions to perform: acquiring a plurality of raw data by detecting, by an X-ray generator, an X-ray irradiated to an object from an X-ray generator a plurality of times; setting at least a part of the object as a region of interest; and reconstructing the tomography image including the region of interest, based on the plurality of raw data, wherein the reconstructing of the tomography image includes: selecting filter kernels based on distances between positions of the X-ray generator that irradiated the X-ray to the region of interest and positions of voxels included in the region of interest; generating filter images by applying the selected filter kernels to the raw data corresponding to the positions of the X-ray generator that irradiated the X-ray; and reconstructing the tomography image from the filter images by back-projecting the filter images.

Before undertaking the DETAILED DESCRIPTION below, it may be advantageous to set forth definitions of certain words and phrases used throughout this patent document: the terms "include" and "comprise," as well as derivatives thereof, mean inclusion without limitation; the term "or," is inclusive, meaning and/or; the phrases "associated with" and "associated therewith," as well as derivatives thereof, may mean to include, be included within, interconnect with, contain, be contained within, connect to or with, couple to or with, be communicable with, cooperate with, interleave, juxtapose, be proximate to, be bound to or with, have, have a property of, or the like; and the term "controller" means any device, system or part thereof that controls at least one operation, such a device may be implemented in hardware, firmware or software, or some combination of at least two of the same. It should be noted that the functionality associated with any particular controller may be centralized or distributed, whether locally or remotely.

Moreover, various functions described below can be implemented or supported by one or more computer programs, each of which is formed from computer readable program code and embodied in a computer readable medium. The terms "application" and "program" refer to one or more computer programs, software components, sets of instructions, procedures, functions, objects, classes, instances, related data, or a portion thereof adapted for implementation in a suitable computer readable program code. The phrase "computer readable program code" includes any type of computer code, including source code, object code, and executable code. The phrase "computer readable medium" includes any type of medium capable of being accessed by a computer, such as read only memory (ROM), random access memory (RAM), a hard disk drive, a compact disc (CD), a digital video disc (DVD), or any other type of memory. A "non-transitory" computer readable medium excludes wired, wireless, optical, or other communication links that transport transitory electrical or other signals. A non-transitory computer readable medium includes media where data can be permanently stored and media where data can be stored and later overwritten, such as a rewritable optical disc or an erasable memory device.

Definitions for certain words and phrases are provided throughout this patent document, those of ordinary skill in the art should understand that in many, if not most instances, such definitions apply to prior, as well as future uses of such defined words and phrases.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 illustrates a structure of a computer tomography (CT) system according to an embodiment of the present disclosure;
FIG. 2 is a diagram for explaining an operation of a CT system acquiring a tomography image, according to an embodiment of the present disclosure;
FIG. 3 is a diagram for explaining an operation of a CT system reconstructing a tomography image, according to an embodiment of the present disclosure;
FIG. 4 is a diagram showing noise non-uniformity existing in a reconstructed tomography image;
FIGS. 5 and 6 are block diagrams of a CT system, according to an embodiment of the present disclosure;
FIGS. 7 and 8 are diagrams illustrating a method, performed by a CT system, of capturing a tomography image in a helical scan manner, according to an embodiment of the present disclosure;
FIG. 9 is a flowchart of a method, performed by a CT system, of reconstructing a tomography image, according to an embodiment of the present disclosure;
FIG. 10 is a flowchart of a method, performed by a CT system, of reconstructing a tomography image, according to an embodiment of the present disclosure;
FIG. 11 is a diagram illustrating a distance between a voxel and each of X-ray generators, according to an embodiment of the present disclosure;
FIG. 12 is a diagram illustrating an example of filter kernels that may be applied to raw data, according to an embodiment of the present disclosure;
FIG. 13 is a diagram illustrating selecting of filter kernels that may be applied to raw data based on filter kernels applied to nearby positioned voxels, according to an embodiment of the present disclosure;
FIG. 14 is a diagram illustrating selecting of filter kernels that may be applied to raw data based on positions of rows included in an X-ray detector, according to an embodiment of the present disclosure; and
FIG. 15 illustrates an example of a tomography image reconstructed by applying a filter kernel, according to an embodiment of the present disclosure.

### DETAILED DESCRIPTION

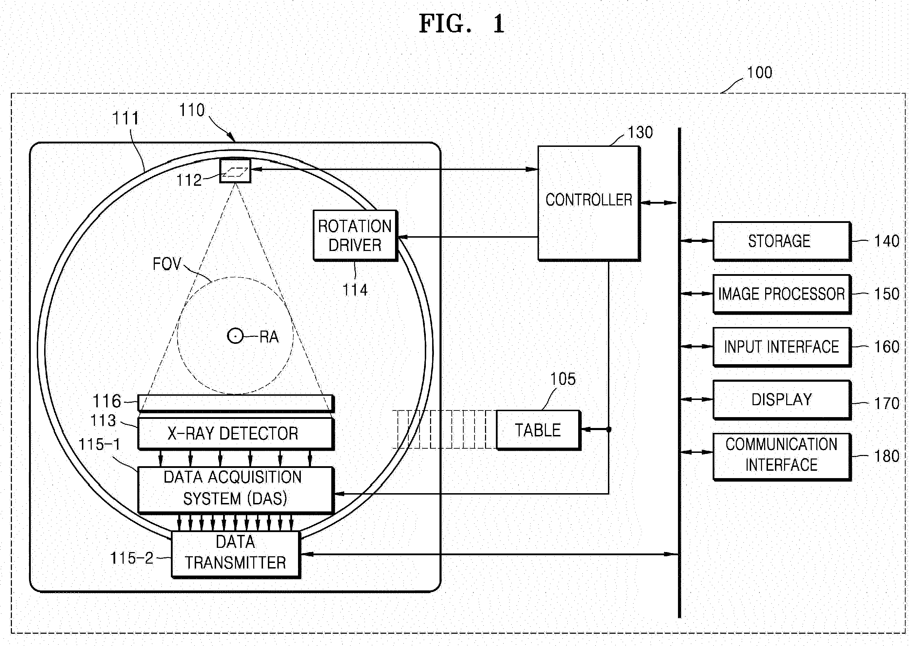

FIGS. 1 through 15, discussed below, and the various embodiments used to describe the principles of the present disclosure in this patent document are by way of illustration only and should not be construed in any way to limit the scope of the disclosure. Those skilled in the art will understand that the principles of the present disclosure may be implemented in any suitably arranged system or device.

The principle of the present disclosure is explained and embodiments are disclosed so that the scope of the present disclosure is clarified and one of ordinary skill in the art to which the present disclosure pertains implements the present disclosure. The disclosed embodiments may have various forms.

Throughout the specification, like reference numerals or characters refer to like elements. In the present specification, all elements of embodiments are not explained, but general matters in the technical field of the present disclosure or redundant matters between embodiments will not be described. Terms 'module' or 'unit' used herein may be implemented using at least one or a combination from among software, hardware, or firmware, and, according to embodiments, a plurality of 'module' or 'unit' may be implemented using a single element, or a single 'module' or 'unit' may be implemented using a plurality of units or elements. The operational principle of the present disclosure and embodiments thereof will now be described more fully with reference to the accompanying drawings.

In the present specification, an image may include a medical image acquired by a medical imaging apparatus, such as a computed tomography (CT) apparatus, a magnetic resonance imaging (MRI) apparatus, an ultrasound imaging apparatus, or an X-ray apparatus.

Throughout the specification, the term 'object' is a thing to be imaged, and may include a human, an animal, or a part of a human or animal. For example, the object may include a part of a body (i.e., an organ), a phantom, or the like.

In the present specification, a 'CT system' or 'CT apparatus' refers to a system or apparatus configured to emit X-rays while rotating around at least one axis relative to an object and photograph the object by detecting the X-rays..

In the specification, a 'CT image' refers to an image constructed from raw data acquired by photographing an object by detecting X-rays that are emitted as the CT system or apparatus rotates about at least one axis with respect to the object.

FIG. 1 illustrates a structure of a CT system 100 according to an embodiment of the present disclosure. The embodiment of the CT system 100 is for illustration only. Other embodiments could be used without departing from the scope of the present disclosure.

In the example shown in FIG. 1, the CT system 100 may include a gantry 110, a table 105, a controller 130, a storage 140, an image processor 150, an input interface 160, a display 170, and a communication interface 180.

The gantry 110 may include a rotating frame 111, an X-ray generator 112, an X-ray detector 113, a rotation driver 114, and a readout device 115.

The rotating frame 111 may receive a driving signal from the rotation driver 114 and rotate around a rotation axis (RA).

An anti-scatter grid 116 may be disposed between an object and the X-ray detector 113 and may transmit most of primary radiation and attenuate scattered radiation. The object may be positioned on the table 105, which may move, tilt, or rotate during a CT scan.

The X-ray generator 112 receives a voltage and a current from a high voltage generator (HVG) to generate and emit X-rays.

The CT system 100 may be implemented as a single-source CT system including one X-ray generator 112 and one X-ray detector 113, or as a dual-source CT system including two X-ray generators 112 and two X-ray detectors 113.

The X-ray detector 113 detects radiation that has passed through the object. For example, the X-ray detector 113 may detect radiation by using a scintillator, a photon counting detector, or the like.

Methods of driving the X-ray generator 112 and the X-ray detector 113 may vary depending on scan modes used for scanning of the object. The scan modes are classified into an axial scan mode and a helical scan mode, according to a path along which the X-ray detector 113 moves. Furthermore, the scan modes are classified into a prospective mode and a retrospective mode, according to a time interval during which X-rays are emitted.

The controller 130 may control an operation of each of the components of the CT system 100. The controller 130 may include a memory configured to store program for performing a function or data and a processor configured to process the program codes or the data. The controller 130 may be implemented in various combinations of at least one memory and at least one processor. The processor may generate or delete a program module according to an operating status of the CT system 100 and processes operations of the program module.

The readout device 115 receives a detection signal generated by the X-ray detector 113 and outputs the detection signal to the image processor 150. The readout device 115 may include a data acquisition system (DAS) 115-1 and a data transmitter 115-2. The DAS 115-1 uses at least one amplifying circuit to amplify a signal output from the X-ray detector 113, and outputs the amplified signal. The data transmitter 115-2 uses a circuit such as a multiplexer (MUX) to output the signal amplified in the DAS 115-1 to the image processor 150. According to a slice thickness or a number of slices, only some of a plurality of pieces of data collected by the X-ray detector 113 may be provided to the image processor 150, or the image processor 150 may select only some of the plurality of pieces of data.

The image processor 150 acquires tomography data from a signal acquired by the readout device 115 (e.g., pure data that is data before being processed). The image processor 150 may pre-process the acquired signal, convert the acquired signal into tomography data, and post-process the tomography data. The image processor 150 may perform some or all of the processes described herein, and the type or order of processes performed by the image processor 150 may vary according to embodiments of the present disclosure.

The image processor 150 may perform pre-processing, such as a process of correcting sensitivity irregularity between channels, a process of correcting a rapid decrease of signal strength, or a process of correcting signal loss due to an X-ray absorbing material, on the signal acquired by the readout device 115.

According to certain embodiments, the image processor 150 may perform some or all of the processes for reconstructing a tomography image, to thereby generate the tomography data. According to an certain embodiments, the tomography data may be in the form of data that has undergone back-projection, or in the form of a tomography image. According to certain embodiments, additional processing may be performed on the tomography data by an external device such as a server, a medical apparatus, or a portable device.

Raw data is a set of data values corresponding to intensities of X-rays that have passed through the object, and may include projection data or a sinogram. The data that has undergone back-projection is acquired by performing back-projection on the raw data by using information about an angle at which X-rays are emitted. The tomography image is acquired by using image reconstruction techniques including back-projection of the raw data.

The storage 140 is a storage medium for storing control-related data, image data, and the like. The storage 140 may include a volatile or non-volatile storage medium.

The input interface 160 receives control signals, data, and the like, from a user. The display 170 may display information indicating an operational status of the CT system 100, medical information, medical image data, and the like.

The CT system 100 includes the communication interface 180 and may be connected to external devices, such as a server, a medical apparatus, and a portable device (smartphone, tablet personal computer (PC), wearable device, and so forth), via the communication interface 180.

In certain embodiments, the communication interface 180 may include one or more components that enable communication with an external device. For example, the communication interface 180 may include a short distance communication module, a wired communication module, and a wireless communication module.

According to embodiments, the CT system 100 may or may not use contrast media during a CT scan. Additionally, in certain embodiments, the CT system 100 may be implemented as a device connected to other equipment.

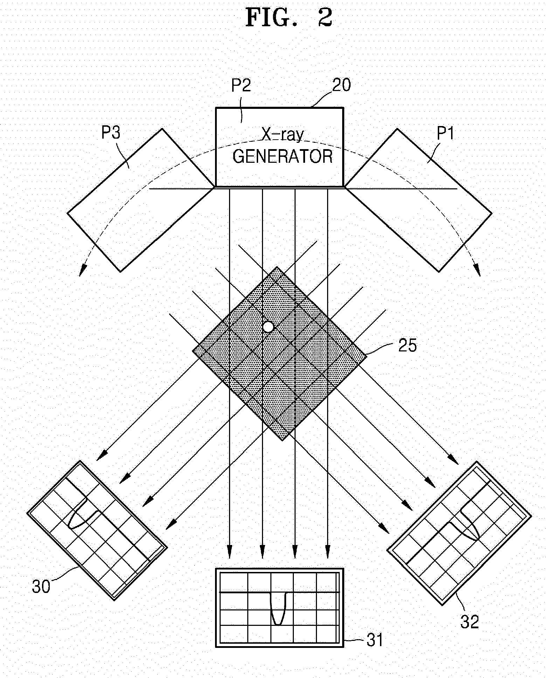

FIG. 2 is a diagram for explaining an operation of a CT system acquiring a tomography image, according to an embodiment of the present disclosure.

Referring to FIG. 2, a CT apparatus may generate raw data by generating an X-ray, irradiating the X-ray to an object 25, and detecting the X-ray transmitted through the object.

Specifically, an X-ray generator 20 included in the CT apparatus may irradiate the object 25 with the X-ray. When the CT apparatus performs CT imaging, the X-ray generator 20 may rotate with respect to the object 25 and acquire a plurality of row data 30, 31, and 32 corresponding to a rotated angle. Specifically, the X-ray generator 20 may detect an X-ray irradiated to the object 25 at a position P1 to acquire the first raw data 30 and detect an X-ray irradiated to the object 25 at a position P2 to acquire the second raw data 31. Then, the X-ray generator 20 may detect an X-ray irradiated to the object 25 at a position P3 to acquire the third raw data 32. In this regard, the first, second, and third raw data 30, 31, and 32 may be projection data.

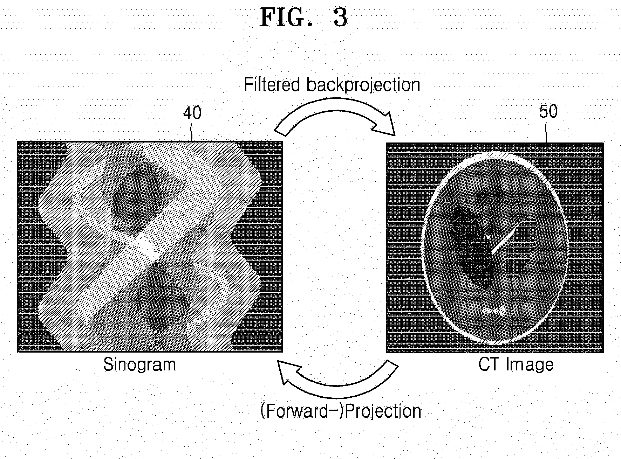

FIG. 3 is a diagram for explaining an operation of a CT system 100 reconstructing a tomography image, according to an embodiment of the present disclosure. The diagram shown in FIG. 3 are for illustration only and other diagrams or images could be used without departing from the scope of this disclosure.

Referring to FIG. 3, the CT system 100 may acquire one sinogram 40 by moving the X-ray generator 20 at a predetermined angular interval and combining the plurality of projection data 30, 31, and 32. The sinogram 40 may be a sinogram acquired by rotating the X-ray generator 20 one cycle and performing CT imaging. The sinogram 40 corresponding to rotation of one cycle may be used for reconstruction of at least one CT image 50. The CT system 100 may acquire the sinogram 40 by performing X-ray imaging while rotating the X-ray generator 20 one cycle (for example, more than half a turn or more than one turn).

The CT system 100 may reconstruct the CT image 50 using the sinogram 40. Specifically, the CT system 100 may reconstruct the CT image 50 by applying a filter kernel to the plurality of projection data 30, 31, and 32 used to acquire the sinogram 40 and performing filtered back-projection on the plurality of projection data 30, 31, and 32.

The CT system 100 may set a region of interest. The region of interest may be set to include a specific organ of the object 10. The CT system 100 may set the region of interest based on a user input to set a specific region of the object 10 as the region of interest. For example, the CT system 100 may set the region of interest based on the user input to set the specific organ of the object 10 as the region of interest.

The CT system 100 may reconstruct a tomography image associated with the region of interest. CT system 100 may reconstruct the tomography image associated with the region of interest by reconstructing each of a plurality of voxels included in the region of interest. The CT system 100 may reconstruct the voxels by applying the filter kernel to raw data and performing filtered back-projection on the raw data.

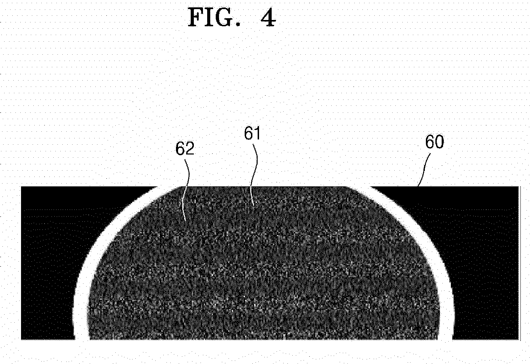

FIG. 4 is a diagram showing noise non-uniformity existing in a reconstructed tomography image according to the related art.

A problem with previous systems is that the reconstructed tomography image according to the related art includes noise non-uniformly. For example, noise may be non-uniformly generated in a zebra pattern in a tomography image 60 as shown in FIG. 4. Specifically, noise may be non-uniformly generated that one partial region 61 of the tomography image 60 is bright and another partial region 62 is dark. In particular, non-uniformly generated noise may be observed in a reconstructed tomography image for an X-ray generator by applying the same filter kernel for raw data acquired by using a helical scan method.

The non-uniform noise included in the tomography image 60 according to the related art makes it difficult for a reader to read the tomography image 60. For example, the non-uniform noise of the zebra pattern may be a problem for the reader to determine brightness of a voxel. Also, the non-uniform noise may make a boundary of internal structures in the tomography image 60 unclear.

Therefore, in order for the reader to make clear reading, there is a need for a plurality of row data to be reconstructed into a tomography image in which noise is generated uniformly.

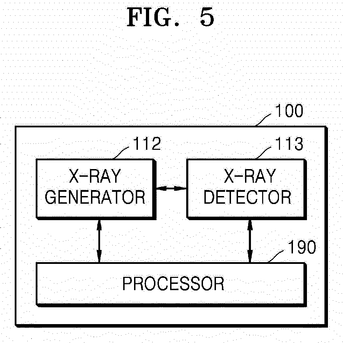

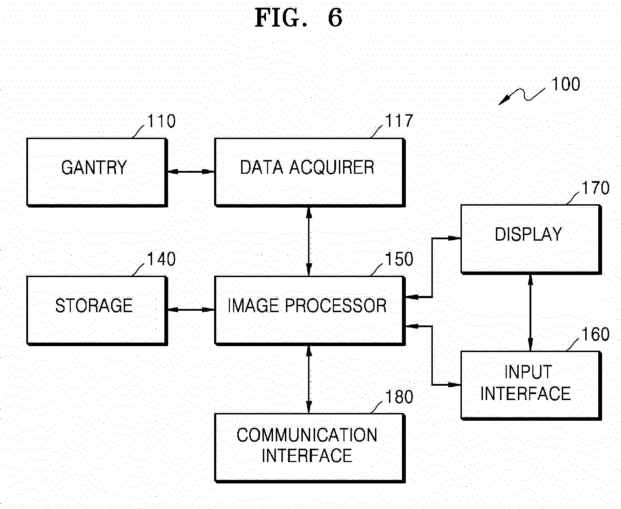

FIGS. 5 and 6 are block diagrams of the CT system 100, according to an embodiment of the present disclosure.

In the example shown in FIG. 5, the CT system 100 may include the X-ray generator 112, the X-ray detector 113, and a processor 190. The CT system 100 may be implemented by more components than the components shown in FIG. 5.

For example, as shown in FIG. 6, the CT system 100 according to some embodiments may include the gantry 110, a data acquirer 117, the storage 140, the image processor 150, the input interface 160, the display 170, and the communication interface 180.

According to certain embodiments, the gantry 110 may include the X-ray generator 112 and the X-ray detector 113. The X-ray generator 112 and the X-ray detector 113 may be positioned to face each other. The gantry 110 has been described above with reference to FIG. 1, and thus a redundant description thereof is omitted.

The data acquirer 117 may include the X-ray generator 112 and the X-ray detector 113. The data acquirer 117 may acquire data when the X-ray detector 113 detects an X-ray irradiated by the X-ray generator 112.

The X-ray generator 112 may irradiate the X-ray to the object 10.

According to certain embodiments, the X-ray generator 112 may rotate around the object 10 and irradiate the X-ray to the object 10 a plurality of times while rotating. For example, the X-ray generator 112 may irradiate the X-ray to the object 10 every time the X-ray generator 112 rotates by a predetermined angle.

According to certain embodiments, the x-ray generator 112 may irradiate the X-ray to the object 10 by using an axial scanning method or a helical scan method.

According to certain embodiments, the X-ray generator 112 may generate information about a location to which the X-ray is irradiated. The X-ray generator 112 may transmit the information about the location to which the X-ray is irradiated to the processor 190.

The X-ray generator 112 has been described above with reference to FIG. 1, and thus a redundant description thereof is omitted.

The X-ray detector 113 may detect the X-ray irradiated from the X-ray generator 112. The X-ray detector 113 may be positioned to face the X-ray generator 112. The X-ray detector 113 may rotate around the object 10 together with the X-ray generator 112. The X-ray detector 113 may detect the X-ray irradiated from the X-ray generator 112 to the object 10 a plurality of times during rotation. The X-ray detector 113 may transmit a detection signal acquired by detecting the X-ray to the processor 190.

The X-ray detector 113 may include an indirect detector detecting and converting the X-ray into light and a direct detector detecting and converting radiation directly into electric charge.

According to certain embodiments, a panel of the X-ray detector 113 may include a plurality of rows. Each of the plurality of rows may detect the irradiated X-ray. The X-ray detector 113 may generate information about a position of each of the plurality of rows. The X-ray detector 113 may transmit the information about the position of each of the plurality of rows to the processor 190. The X-ray detector 113 may transmit a detection signal acquired by detecting the X-ray from each of the plurality of rows to the processor 190. The X-ray detector 113 has been described above with reference to FIG. 1, and thus a redundant description thereof is omitted.

The data acquirer 117 may include a data acquisition system (DAS). The DAS may be connected to the X-ray detector 113. That is, the data acquirer 117 may collect an electric signal generated by the X-ray detector 113 by wired or wirelessly. The electrical signal generated by the X-ray detector 113 may be provided to an analog/digital converter (not shown) via an amplifier (not shown).

The data acquirer 117 may provide only partial data acquired from the X-ray detector 113 to the image processor 150 according to a slice thickness or the number of slices. Alternatively, the image processor 150 may select only partial data.

The data acquirer 117 may acquire a plurality of raw data based on the X-ray detected while the X-ray detector 113 rotates around the object 10. In this regard, the raw data may be projection data acquired by irradiating radiation to an object or a sinogram that is a set of projection data. When the X-ray generator 112 irradiates the X-ray to the object 10 at a predetermined position, a viewpoint or a direction at which the X-ray generator 112 views the object 10 may be referred to as a view. The projection data may be raw data acquired in correspondence to one view. The sinogram means raw data acquired by sequentially arranging a plurality of projection data.

The data acquirer 117 may transmit the acquired raw data to the image processor 150.

The processor 190 may be configured as one or more physical processors. The processor 190 may also be fabricated in the form of a dedicated hardware chip or may be fabricated as part of a general purpose processor according to the related art (e.g., a CPU or an application processor) or a graphics specific processor (e.g., a GPU).

The processor 190 may perform functions of the controller 130 and the image processor 150. In particular, the processor 190 may perform a function of the controller 130 that controls the overall operation of the CT system 100. Also, the processor 190 may perform a function of the image processor 150 that performs image processing on the raw data.

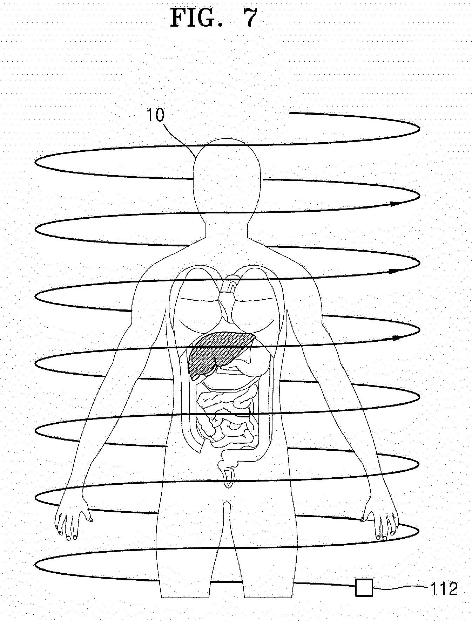

The controller 130 may control the overall operation of the CT system 100. For example, the controller 130 may generally control the gantry 110, the X-ray generator 112, the X-ray detector 113, the data acquirer 117, the storage 140, the image processor 150, the input interface 160, the display 170, and the communication interface 180 by executing programs stored in the storage 140. Also, the processor 190 may perform a function of the CT system 100 described with reference to in FIGS. 7 to 14 by executing the programs stored in the storage 140.

The storage 140 may store data acquired according to tomography imaging. Specifically, the storage 140 may store at least one of the projection data which is the raw data and the sinogram. Also, the storage 140 may store various data, programs, and the like necessary for reconstruction of a tomography image and may store a finally reconstructed tomography image.

The storage 140 may include at least one storage medium of be a flash memory type, a hard disk type, a multimedia card micro type, a card type memory (SD, XD memory, or the like), a RAM (Random Access Memory), SRAM (Static Random Access Memory), ROM (Read Only Memory), EEPROM (Electrically Erasable Programmable Read-Only Memory), PROM (Programmable Read-Only Memory), magnetic memory, magnetic disc, and optical disc.

The image processor 150 may perform image processing on the raw data received from the data acquirer 117. For example, pre-processing, conversion into tomography image data, and post-processing on the tomography image data may be performed. For example, pre-processing may include a sensibility non-uniformity correction process between channels, a loss correction process of a signal due to a sharp reduction in signal intensity or a metal-like X-ray absorber.

Input data of the image processor 150 may be referred to as raw data or projection data. The projection data may be a set of data values corresponding to the intensity of X-rays transmitted through the object 10. Such raw data may be stored in the storage 124 together with imaging conditions (e.g., tube voltage, irradiation angle, etc.) upon acquisition of data.

The image processor 150 may set at least a part of the object 10 as a region of interest. The image processor 150 may reconstruct the tomography image by reconstructing voxels included in the region of interest based on the plurality of raw data. For example, the image processor 150 may reconstruct the voxels by accumulating and back-projecting the plurality of raw data related to the voxels included in the region of interest. The image processor 150 may generate a filter image by applying a filter kernel to at least one of the plurality of raw data, and reconstruct the voxels by back-projecting the generated filter image.

The image processor 150 may select the filter kernel to be used for reconstructing the voxels based on distances between a position of the X-ray generator 112 irradiating the X-ray to the region of interest and a position of each of the voxels included in the region of interest.

According to certain embodiments, the image processor 150 may select the filter kernel to be used to reconstruct a first voxel, based on a distance between a position of a first voxel (a reconstructing voxel) and a position to which the X-ray generator 112 irradiates the X-ray. For example, the image processor150 may select a filter kernel having a smaller noise as a distance between the position of the X-ray generator 112 irradiating the X-ray and the first voxel is closer. The image processor 150 may select a filter kernel having a smaller noise as the distance between the position of the X-ray generator 112 irradiating the X-ray and the first voxel is farther.

According to certain embodiments, the image processor 150 selects the filter kernel based on at least one of the longest distance and the shortest distance among the distances between positions of the X-ray generator 112 irradiating the X-ray and the position of the first voxel. Specifically, the image processor150 may select the filter kernel by comparing at least one of the longest distance and the shortest distance with the distances between positions of the X-ray generator 112 irradiating the X-ray and the position of the first voxel.

According to certain embodiments, the image processor 150 may select the filter kernel to be used to reconstruct the first voxel by referring to a second voxel (already reconstructed voxel) located near the first voxel (reconstructing voxel). For example, the image processor 150 may select the filter kernel to be used to reconstruct the first voxel based on the filter kernel selected when reconstructing the second voxel. The image processor 150 may select a filter kernel to be applied to raw data acquired by using a first row, based on a position of the first row which detects the X-ray transmitted through the voxel among a plurality of rows included in the X-ray detector 113. The image processor 150 may select a plurality of filter kernels based on a distance between the position of the first voxel (reconstructing voxel) and a first position of the X-ray generator 112 irradiating the X-ray. The image processor150 may generate a plurality of first filter images by applying each of the plurality of filter kernels to the raw data acquired using the X-ray irradiated by the X-ray generator 112 at the first position. The image processor 150 may generate a second filter image by combining the plurality of filter images. The image processor150 may reconstruct the first voxel by back-projecting the generated second filter image.

The image processor150 may select at least one filter kernel among a plurality of predetermined filter kernels. The image processor150 may generate a plurality of first filter images by applying the selected filter kernel to the plurality of raw data. The image processor150 may generate a second filter image by applying a modulation filter to the generated first filter images. The image processor 150 may reconstruct the voxel by back-projecting the second filter image. The modulation filter may be a filter for modulating characteristics (for example, noise, sharpness) of a filter image and may include a smoothing filter reducing noise, a Gaussian filter, a filter increasing noise and sharpness. The modulation filter may include a plurality of filters having different degrees of varying noise and sharpness. The image processor 150 may select the modulation filter based on the distance between the position of the first voxel (reconstructing voxel) and the position of the X-ray generator 112 irradiating the X-ray.

The image processor 150 may select at least one from the filter kernels included in a set of filter kernels. The set of filter kernels may have predetermined noise and sharpness. The set of filter kernels may be plural. The image processor 150 may select at least one from filter kernels included in a set of filter kernels selected by a user from the plurality of sets of filter kernels and apply the selected filter kernel to the raw data.

The image processor150 may generate a plurality of filter kernels that are slightly different in characteristics (for example, noise, sharpness) of a filter kernel by applying a plurality of kernel modulation filters (for example, filters varying noise and sharpness of the filter kernel) to one filter kernel. The generated plurality of filter kernels may include noise and sharpness similar to those of the filter kernel to which the modulation filters are applied. The image processor150 may select a filter kernel to be used for reconstructing the voxels based on distances between the positions of the X-ray generator 112 irradiating the X-ray and positions of the respective voxels included in the region of interest.

The input interface 160 may receive an external input with respect to an X-ray imaging condition, an image processing condition, and the like. For example, the X-ray imaging condition may include a plurality of tube voltages, setting of energy values of a plurality of X-rays, selection of an imaging protocol, selection of an image reconstruction method, setting of a FOV region, a slice number, a slice thickness, setting of image post-processing parameters, and the like. The image processing condition may include resolution of an image, setting of an attenuation coefficient of the image, setting of a combination ratio of the image, and the like.

The input interface 160 may include a device or the like for receiving a predetermined input from outside. For example, the input interface 160 may include a microphone, a keyboard, a mouse, a joystick, a touch pad, a touch pen, a voice, and a gesture recognition device, etc.

The display 170 may display the tomography image reconstructed by the image processor 150. Also, the display 170 may display a user interface screen necessary for performing tomography.

The communication interface 180 may transmit and receive data, power, and the like among the above-described elements using at least one of wired, wireless, and optical communication.

The communication interface 180 may perform communication with an external device, an external medical device, or the like through a server (not shown) or the like. The communication interface 180 may be connected to a network by wired or wirelessly to perform communication with an external device such as a server (not shown), an external medical device (not shown), or a portable device (not shown), etc. The communication interface 180 may exchange data with a hospital server connected through a PACS (Picture Archiving and Communication System) or other medical apparatuses in a hospital. Also, the communication interface 180 may perform data communication with an external device or the like according to the DICOM (Digital Imaging and Communications in Medicine) standard.

The communication interface 180 may transmit and receive data related to a diagnosis of the object 10 through the network. The communication interface 180 may also transmit and receive medical images and the like acquired from other medical devices such as an MRI apparatus and an X-ray imaging apparatus.

The communication interface 180 may receive a diagnosis history or a treatment schedule of a patient from a server (not shown) and may utilize the same for clinical diagnosis of the patient. The communication interface 180 may perform data communication with a server (not shown) or a medical device (not shown) in a hospital, as well as with a portable device (not shown) of the user or the patient.

When the disclosed embodiments of the method for generating a tomography image are implemented in a software module (or a program module including an instruction), the software module may be stored in non-transitory computer readable media. Further, in this case, at least one software module may be provided by an operating system (OS) or by a predetermined application. Alternatively, some of the at least one software module may be provided by the OS, and others may be provided by the predetermined application.

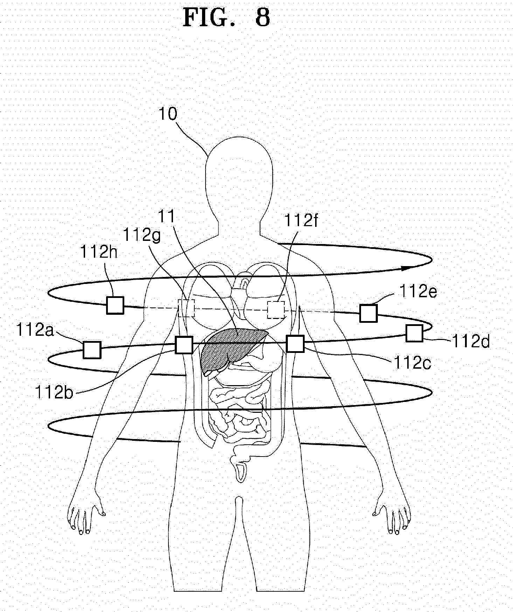

FIGS. 7 and 8 are diagrams illustrating a method, performed by the CT system 100, of capturing a tomography image in a helical scan manner, according to an embodiment.

Referring to FIGS. 7 and 8, the CT system 100 may irradiate an X-ray to the object 10 using the X-ray generator 112 moving in one direction while rotating around the object 10. That is, the X-ray generator 112 may irradiate the X-ray to the object 10 at predetermined intervals while moving the object 10 helically along an axis.

Referring to FIG. 8, the CT system 100 may irradiate the X-ray to a part 11 of the object 10 while rotating the X-ray generator 112 around the part 11 of the object 10, to acquire raw data for reconstructing the tomography image of the part 11 of the object 10. For example, the X-ray generator 112 may irradiate the X-ray to the part 11 of the object 10 while rotating around the part 11 of the object 10 for more than half a cycle.

According to an embodiment, the CT system 100 may acquire information about a position of the X-ray generator 112 when the X-ray generator 112 irradiates the X-ray to the object 10.

For example, the CT system 100 may acquire the information about the position of the X-ray generator 112 using absolute position coordinates. Specifically, the CT system 100 may acquire the information about the position of the X-ray generator 112 by generating a three-dimensional position coordinate system based on vertexes of a table 105 and detecting which part of the three-dimensional position coordinate system corresponding to the X-ray generator 112.

In another example, the CT system 100 may acquire the information about the position of the X-ray generator 112 using relative position coordinates. Specifically, the CT system 100 may acquire the information about the position of the X-ray generator 112 by detecting in which part the X-ray generator 112 is located with respect to a specific position (for example, an organ such as liver, stomach, or heart) of the object 10. Alternatively, the CT system 100 may acquire the information about the position of the X-ray generator 112 by detecting in which part the X-ray generator 112 is located with respect to a specific position of the table 105 (for example, the center of the table 105).

Referring to FIG. 8, to acquire data related to the part 11 of the object 10, the X-ray generator 112 may irradiate the X-ray to the part 11 of the object 10 a plurality of times while rotating around the part 11 of the object 10 one turn. In the example shown in FIG. 8, it is assumed that the X-ray generator 112 irradiates the x-ray 8 times while rotating the part 11 of the object 10 one turn. However, the number of times the X-ray is irradiated is not limited thereto.

Referring to FIG. 8, the X-ray generator 112 may move in one direction while rotating around the object 10 to irradiate the X-ray to the part 11 of the object 10 at predetermined cycles.

More specifically, each of a X-ray generator 112a at a first time point, a X-ray generator 112b at a second time point, a X-ray generator 112c at a third time point, a X-ray generator 112d at a fourth time point, a X-ray generator 112e at a fifth time point, a X-ray generator 112f at a sixth time point t, a X-ray generator 112g at a seventh time point, and a X-ray generator 112h at an eighth time point may generate the X-ray to the object 10. The position of the x-ray generator 112 may be at the same position as the X-ray generator 112h at the eighth time point, when the X-ray generator 112a rotates one turn.

The X-ray detector 113 positioned to face the X-ray generator 112 may detect the X-ray irradiated from the X-ray generator 112 while rotating around the object 10 together with the X-ray generator 112. The X-ray detector 113 may acquire raw data corresponding to positions of the X-ray generators 112a, 112b, 112c, 112d, 112e, 112f, 112g, and 112hat the first time point to the eighth time point by detecting the X-ray irradiated from the X-ray generators 112a, 112b, 112c, 112d, 112e, 112f, 112g, and 112h.at the first time point to the eighth time point. That is, the X-ray detector 113 may acquire the raw data corresponding to each of the first through eighth time points.

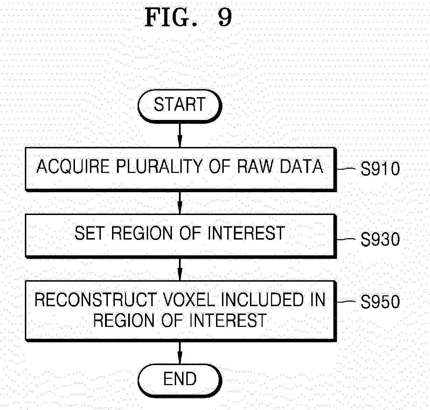

FIG. 9 is a flowchart of a method, performed by the CT system 100, of reconstructing a tomography image, according to an embodiment of the present disclosure.

Referring to FIG. 9, the CT system 100 may acquire a plurality of raw data related to the object 10 (S910), set at least a part of the object 10 as a region of interest (S930), and reconstruct a first voxel included in the region of interest, thereby reconstructing the tomography image (S950).

Referring to operation S910, the CT system 100 may acquire the plurality of raw data by detecting X-rays irradiated from the X-ray generator 112 to the object 10 a plurality of times by using the X-ray detector 113. The plurality of raw data may include projection data or a sinogram. The method, performed by the X-ray generator 112, of irradiating the X-rays to the object 10 a plurality of times and acquiring the plurality of raw data are described above, and thus redundant descriptions thereof are omitted.

Referring to operation S930, the CT system 100 may set the region of interest in at least a part of the object 10. For example, the CT system 100 may set the region of interest in the part (e.g., an internal organ such as liver, heart, or stomach) of the object 10. The CT system 100 may set the region of interest in at least a part of the object 10 based on an input that sets a region of interest received from a user.

Referring to operation S950, the CT system 100 may reconstruct the tomography image by reconstructing voxels contained in the region of interest based on the plurality of raw data acquired in operation S910.

According to certain embodiments, the CT system 100 may reconstruct the voxels by back-projecting the plurality of raw data. The CT system 100 may select a filter kernel to be applied to at least one of the plurality of raw data. Same or different filter kernels may be applied to each of the plurality of raw data. That is, the CT system 100 may select the filter kernel to be applied to each of the plurality of raw data based on characteristics of each of the plurality of raw data. For example, the CT system 100 may select the filter kernel based on a distance between a position of a voxel being reconstructed and a position of an X-ray generator irradiating an X-ray.

According to certain embodiments, the CT system 100 may generate a filter image by applying the filter kernel to each of the plurality of back-projected raw data.

According to certain embodiments, the CT system 100 may reconstruct the voxels by back-projecting the generated filter images.

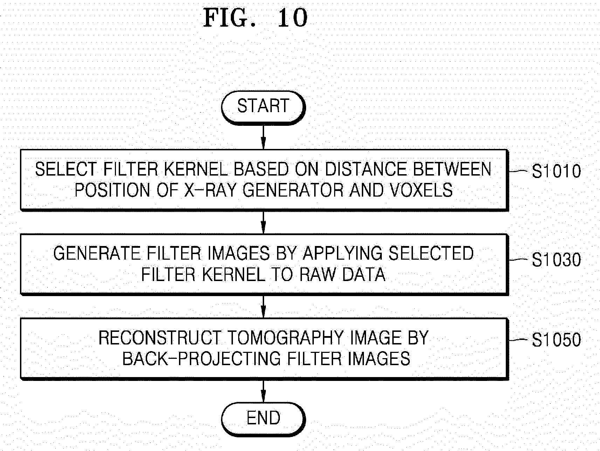

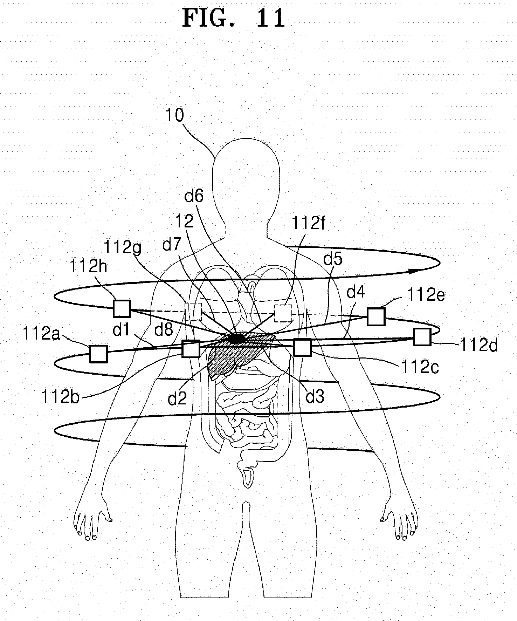

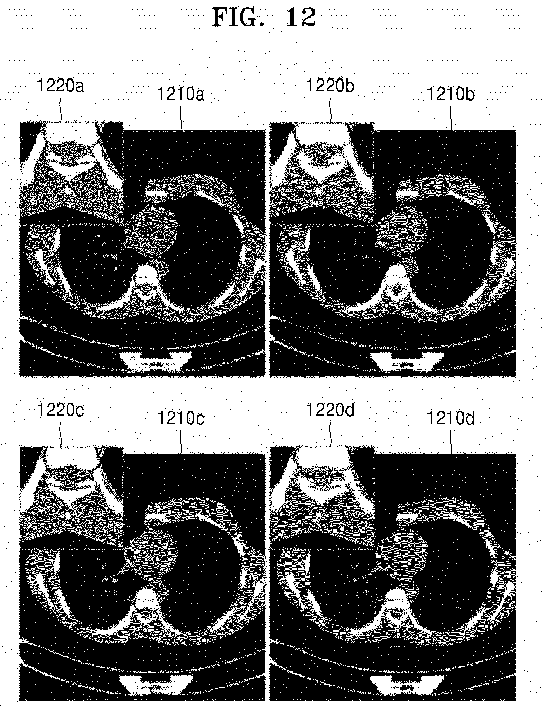

FIG. 10 is a flowchart of a method, performed by the CT system 100, of reconstructing a tomography image, according to an embodiment of the present disclosure. FIG. 11 is a diagram illustrating a distance between a voxel 12 and each of the X-ray generators 112a, 112b, 112c, 112d, 112e, 112f, 112g, and 112h, according to an embodiment of the present disclosure. FIG. 12 is a diagram illustrating an example of filter kernels that may be applied to raw data, according to an embodiment of the present disclosure.

Referring to FIG. 10, to reconstruct the tomography image, the CT system 100 may select a filter kernel to be applied to at least one raw data based on a distance between a position of an X-ray generator and voxels included in a region of interest (S1010), generate filter images by applying the selected filter kernel to at least one raw data (S1030), and reconstruct the tomography image by back-projecting the generated filter images (S1050).

Referring to operation S1010, the CT system 100 may select the filter kernel based on the distance between the position of the X-ray generator irradiating an X-ray to the region of interest and the voxel included in the region of interest.

According to certain embodiments, the CT system 100 may select the filter kernel to be used to reconstruct the voxel based on a user input.

According to certain embodiments, the CT system 100 may select the filter kernel to be applied to each of the raw data by an algorithm.

According to certain embodiments, the CT system 100 may select at least one filter kernel applied to each of the raw data corresponding to each position to which the X-ray generator irradiates the X-ray based on distances between the voxel and each position to which the X-ray generator irradiates the X-ray. The raw data corresponding to the position that the X-ray generator irradiates the X-ray may include the raw data acquired by detecting the irradiated X-ray data. The CT system 100 may select at least one filter kernel from filter kernels having predetermined noise and sharpness.

Referring to FIG. 11, the CT system 100 may set a liver, which is a part of the object 10, as the region of interest. The CT system 100 may acquire information about each of positions of the X-ray generators 112a, 112b, 112c, 112d, 112e, 112f, 112g, and 112h irradiating the X-rays to the region of interest.

Also, the CT system 100 may acquire information about distances d1, d2, d3, d4, d5, d6, d7 and d8 between the voxel 12 included in the region of interest and positions of the X-ray generators 112a, 112b, 112c, 112d, 112e, 112f, 112g, and 112h of first through eighth time points.

The CT system 100 may select a filter kernel applied to each of raw data corresponding to the first through eighth time points based on the distances d1, d2, d3, d4, d5, d6, d7 and d8 between the voxel 12 and the positions of the X-ray generators 112a, 112b, 112c, 112d, 112e, 112f, 112g, and 112h.

Referring to FIG. 12, the CT system 100 may select the filter kernels that may be applied to the raw data. The filter kernels may differ in a degree of noise involved. For example, there may be filter kernels 1210a and 1220a with very high noise, filter kernels 1210c and 1220c with high noise, filter kernels 1210b and 1220b with low noise, and filter kernels 1210d and 1220d with little noise. FIG. 12 shows an example of four filter kernels but is not limited thereto. A larger number of filter kernels may be selected.

According to certain embodiments, the CT system 100 may select a set of filter kernels from a plurality of sets of filter kernels. Each of the plurality of sets of filter kernels may have predetermined noise and sharpness. Each of the plurality of sets of filter kernels may include different characteristics. For example, characteristics included in the set of filter kernels may include an amount of noise, a kind of noise, an expression pattern of noise, a form of noise, and the like. Also, the characteristics included in the set of filter kernels may include a degree of sharpness, a degree of anti-aliasing, an anti-aliasing algorithm, and the like. A plurality of filter kernels included in each of the plurality of sets of filter kernels may include the characteristics included in each of the plurality of filter kernel sets. Each of the plurality of sets of filter kernels may include at least one filter kernel having predetermined noise and sharpness. The CT system 100 may select at least one filter kernel included in the selected set of filter kernels to apply to the raw data.

According to certain embodiments, the CT system 100 may generate a plurality of filter kernels by applying a plurality of kernel modulation filters to one of pre-generated filter kernels and select at least one of the generated plurality of filter kernels. The generated plurality of filter kernels may include characteristics similar to those of the filter kernel to which the kernel modulation filter are applied.

The kernel modulation filter may be filter for modulating the characteristics of the filter kernel and may be a smoothing filter, a Gaussian filter, or a filter increasing noise and sharpness, and so forth. Each of the plurality of kernel modulation filters may be a plurality of filters having different degrees of varying noise and sharpness.

Each of the pre-generated filter kernels may include different characteristics. The characteristics included in the pre-generated filter kernels may include an amount of noise, a type of noise, an expression pattern of noise, a form of noise, and the like. Also, the characteristics included in the pre-generated filter kernels may include a degree of sharpness, a degree of anti-aliasing, an anti-aliasing algorithm, and the like.

According to certain embodiments, the CT system 100 may select at least one filter kernel applied to each of raw data corresponding to each of positions of the X-ray generators 112a, 112b, 112c, 112d, 112e, 112f, 112g, and 112h based on the longest and shortest distances among the distances between the voxel 12 and the positions of the X-ray generators 112a, 112b, 112c, 112d, 112e, 112f, 112g, and 112h of first through eighth time points.

According to certain embodiments, the CT system 100 may select at least one filter kernel among the plurality of filter kernels having different degrees of noise applied to each of the raw data based on the distances d1, d2, d3, d4, d5, d6, d7 and d8 between the voxel 12 and the X-ray generators 112a, 112b, 112c, 112d, 112e, 112f, 112g, and 112h. For example, the CT system 100 may select a filter kernel having a small amount of noise as the distances d1, d2, d3, d4, d5, d6, d7 and d8 between the voxel 12 and the X-ray generators 112a, 112b, 112c, 112d, 112e, 112f, 112g, and 112h are closer to each other. Also, the CT system 100 may select a filter kernel having a great amount of noise as the distances d1, d2, d3, d4, d5, d6, d7 and d8 between the voxel 12 and the X-ray generators 112a, 112b, 112c, 112d, 112e, 112f, 112g, and 112h are farther from each other.

Referring to FIG. 11, the CT system 100 may select a filter kernel applied to each of the raw data based on the shortest distance d7 and the longest distance d4.

According to certain embodiments, the CT system 100 may select filter kernels applied to the raw data of the first through eighth time points by comparing the shortest distance d7 with each of the distances d1, d2, d3, d4, d5, d6, d7 and d8. The CT system 100 may determine a ratio between the shortest distance d7 with each of the distances d1, d2, d3, d4, d5, d6, d7 and d8 and select a filter kernel corresponding to the ratio.

For example, when the ratio of the distance d5 and the shortest distance d7 at the fifth time point is 1.2, the CT system 100 may select filter kernels corresponding to the distance d5 between the X-ray generator 112e at the fifth time point and the voxel 12 as the filter kernels 1210c and 1220c shown at the bottom left of FIG. 12.

The CT system 100 may determine the ratio between each of the distances d1, d2, d3, d4, d5, d6, d7 and d8 of the first to eighth time points and the shortest distance d7 linear-proportionally. Alternatively, the CT system 100 may determine the ratio between each of the distances d1, d2, d3, d4, d5, d6, d7 and d8 of the first to eighth time points and the shortest distance d7 in proportional to a log scale. Alternatively, the CT system 100 may determine the ratio between each of the distances d1, d2, d3, d4, d5, d6, d7 and d8 of the first to eighth time points and the shortest distance d7 in proportional to an exponential function.

According to certain embodiments, the CT system 100 may select the filter kernels applied to the raw data of the first through eighth time points by comparing the longest distance d4 with each of the distances d1, d2, d3, d4, d5, d6, d7 and d8. The CT system 100 may determine the ratio between each of the distances d1, d2, d3, d4, d5, d6, d7 and d8 of the first through eighth time points and the longest distance d4 and select a filter kernel corresponding to the ratio.

For example, when the distance d5 of the fifth viewpoint and the longest distance d4 is 0.6, the CT system 100 may select filter kernels corresponding to the distance d5 between the X-ray generator 112e at the fifth time point and the voxel 12 as the filter kernels 1210c and 1220c shown at the bottom left of FIG. 12.

The CT system 100 may determine the ratio between each of the distances d1, d2, d3, d4, d5, d6, d7 and d8 of the first to eighth time points and the longest distance d4 linear-proportionally. Alternatively, the CT system 100 may determine the ratio between each of the distances d1, d2, d3, d4, d5, d6, d7 and d8 of the first to eighth time points and the longest distance d4 in proportional to a log scale. Alternatively, the CT system 100 may determine the ratio between each of the distances d1, d2, d3, d4, d5, d6, d7 and d8 of the first to eighth time points and the longest distance d4 in proportional to an exponential function.

According to certain embodiments, the CT system 100 may select a filter kernel applied to each of the raw data based on the ratio of the shortest distance d7 and the longest distance d4. When n filter kernels are applied to the raw data, the CT system 100 may divide a distance between the shortest distance d7 and the longest distance d4 into n steps. The CT system 100 may set a filter kernel corresponding to each step. The CT system 100 may determine which step among steps between the shortest distance d7 and the longest distance d4 corresponds to each of the distances d1, d2, d3, d4, d5, d6, d7 and d8 between the voxel 12 and the positions of the X-ray generators 112a, 112b, 112c, 112d, 112e, 112f, 112g, and 112h of first through eighth time points.

For example, the CT system 100 may select a filter kernel corresponding to a step corresponding to the distance d5 between the X-ray generator 112e at the fifth time point and the voxel 12 as the filter kernel to be applied to the raw data of the fifth time point. The CT system 100 may divide the distance between the shortest distance d7 and the longest distance d4 into n steps linear-proportionally. Alternatively, the CT system 100 may divide the distance between the shortest distance d7 and the longest distance d4 into n steps in proportional to a log scale. Alternatively, the CT system 100 may divide the distance between the shortest distance d7 and the longest distance d4 into n steps exponential-proportionally.

According to certain embodiments, the filter kernel corresponding to each step may be set based on noise included in each filter kernel. The filter kernel corresponding to the shortest distance d7 may be a filter kernel having the least noise. The filter kernel corresponding to the longest distance d4 may be a filter kernel having the largest amount of noise. Filter kernels respectively including gradually increasing amounts of noise may respectfully correspond to stages from the shortest distance d7 to the longest distance d4.

According to certain embodiments, the CT system 100 may select filter kernels corresponding to steps, among the n steps, corresponding to the distances d1, d2, d3, d4, d5, d6, d7 and d8 between the voxel 12 and the positions of the X-ray generators 112a, 112b, 112c, 112d, 112e, 112f, 112g, and 112h of first through eighth time points.

For example, the CT system 100 may select the filter kernels 1210a and 1220a shown in the top left of FIG. 12 as filter kernels corresponding to the longest distance d4. The CT system 100 may select the filter kernels corresponding to the shortest distance d7 as the filter kernels 1210d and 1220d shown at the bottom right of FIG. 12. The CT system 100 may select the filter kernels corresponding to the distance d5 between the X-ray generator 112e at the fifth time point and the voxel 12 as the filter kernels 1210b and 1220b shown at the top right of FIG. 12.

According to certain embodiments, the CT system 100 may select two or more filter kernels to be applied to each of the raw data corresponding to each of the first through eighth time points based on the distances d1, d2, d3, d4, d5, d6, d7 and d8 between the voxel 12 and the positions of the X-ray generators 112a, 112b, 112c, 112d, 112e, 112f, 112g, and 112h of first through eighth time points. For example, the CT system 100 may select filter kernels applied to the raw data corresponding to the fifth time point as the filter kernels 1210c and 1220c shown at the bottom left of FIG. 12 and the filter kernels 1210b and 1220b shown at the top right of FIG. 12.

Referring to operation S1030, the CT system 100 may generate the filter images by applying the selected filter kernel to the at least one raw data.

According to certain embodiments, the CT system 100 may generate the filter images by applying the selected filter kernel to each of the raw data corresponding to each position to which the X-ray generator irradiates the X-ray.

According to certain embodiments, the CT system 100 may generate first filter images by applying two or more selected filter kernels to each of the raw data. Also, the CT system 100 may generate a second filter image by combining the first filter images. For example, the CT system 100 may generate the first filter image by applying the filter kernels 1210c and 1220c shown in the bottom left of FIG. 12 to the raw data corresponding to the fifth time point. Also, the CT system 100 may generate the first filter image by applying the filter kernels 1210d and 1220d shown in the bottom right of FIG. 12 to the raw data corresponding to the fifth time point. The CT system 100 may generate the second filter image by combining the generated two or more first filter images. The CT system 100 may generate the second filter image by combining the first filter images such that a degree of noise included in the second filter image corresponds to the middle of a degree of noise included in the first filter images.

According to certain embodiments, the CT system 100 may adjust a combination ratio of the first filter images based on a distance between the positions of the X-ray generator and a voxel. Specifically, the CT system 100 may adjust the combination ratio of the first filter images generated by applying the filter kernels 1210c and 1220c shown in the bottom left of FIG. 12 and the filter kernels 1210d and 1220d shown in the bottom right of FIG. 12 to the raw data corresponding to the fifth time point based on the distance d5 corresponding to the fifth time point. The CT system 100 may adjust the combination ratio of the first filter images such that the degree of noise included in the second filter image is closer to the degree of noise included in the first filter image generated by applying the filter kernels 1210c and 1220c shown in the bottom left of FIG. 12 to the raw data corresponding to the fifth time point.

Referring to operation S1050, the CT system 100 may reconstruct the tomography image by back-projecting the generated filter images. The CT system 100 may back-project the second filter image. Also, the CT system 100 may back-project a plurality of first filter images.

The generated tomography image may have uniform noise. Thus, the user can read the generated tomography image clearly.

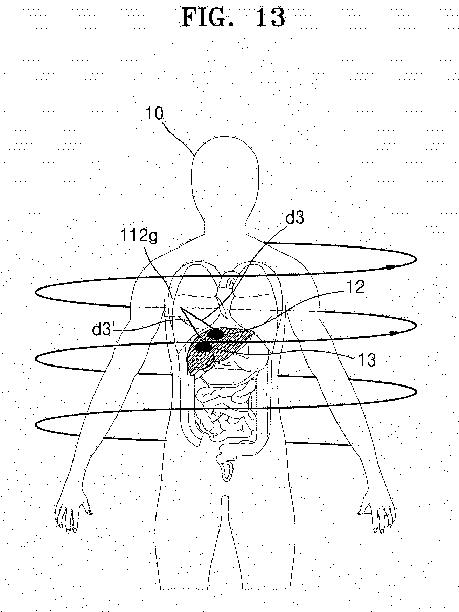

FIG. 13 is a diagram illustrating selecting filter kernels that may be applied to raw data based on filter kernels applied to nearby positioned voxels, according to an embodiment of the present disclosure.

Referring to FIG. 13, the CT system 100 may reconstruct a tomography image by reconstructing voxels included in a region of interest. The region of interest may include a first voxel 12 and a second voxel 13. In this regard, the first voxel 12 may represent a currently reconstructed voxel, and the second voxel 13 may represent a reconstructed voxel. The first voxel 12 and the second voxel 13 may be positioned adjacent to each other. For example, the second voxel 13 may be located within a predetermined distance from the first voxel 12. The first voxel 12 and the second voxel 13 may be located between a predetermined number of voxels or less.

According to certain embodiments, the CT system 100 may select a filter kernel to be used to reconstruct the first voxel 12, based on a filter kernel selected when reconstructing the second voxel 13. For example, the CT system 100 may select a filter kernel to be applied to raw data corresponding to a third time point when reconstructing the first voxel 12 based on a filter kernel applied to raw data corresponding to the third time point when reconstructing the second voxel 13.

Since the first voxel 12 is located near the second voxel 13, the first distance d3 between a position of the X-ray generator 112g irradiating an X-ray at the third time point and the first voxel 12 may be similar to a second distance d3' between the position of the X-ray generator 112g irradiating the X-ray at the third time point and the second voxel 13. When the CT system 100 applies the filter kernels 1210c and 1220c shown in the bottom left of FIG. 12 to the raw data corresponding to the third time point when reconstructing the second voxel 13, the CT system 100 may select filter kernels applied to the raw data corresponding to the third time point as the filter kernels 1210c and 1220c shown in the bottom left of FIG. 12.

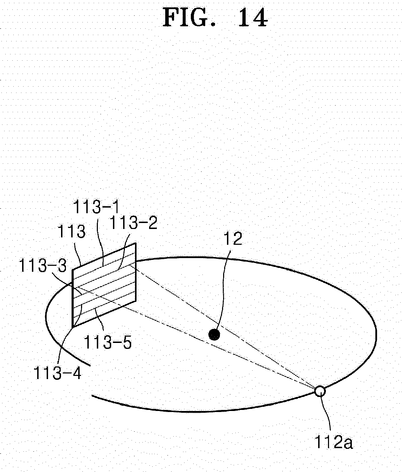

FIG. 14 is a diagram illustrating selecting filter kernels that may be applied to raw data based on positions of rows included in the X-ray detector 113, according to an embodiment of the present disclosure.

Referring to FIG. 14, the X-ray detector 113 may be positioned to face the X-ray generator 112. The X-ray detector 113 may include a plurality of rows 113-1, 113-2, 113-3, 113-4, and 113-5. For the sake of explanation, FIG. 14 shows five rows, but the disclosure is not limited thereto.

The X-ray generator 112 may irradiate an X-ray to a part of an object corresponding to a voxel. Each of the rows 113-1, 113-2, 113-3, 113-4, and 113-5 included in the X-ray detector 113 may detect the irradiated X-ray. The CT system 100 may acquire the raw data corresponding to each of the rows 113-1, 113-2, 113-3, 113-4, and 113-5 based on the X-ray detected by each of the rows 113-1, 113-2, 113-3, 113-4, and 113-5 included in the X-ray detector 113.

According to certain embodiments, the row 113-2 included in the X-ray detector 113 may detect an X-ray transmitted through the voxel 12. That is, the CT system 100 may acquire raw data corresponding to the X-ray transmitted through the voxel 12 using the row 113-2 included in the X-ray detector 113.

According to certain embodiments, the CT system 100 may acquire information about a position of each of the rows 113-1, 113-2, 113-3, 113-4, and 113-5 included in the X-ray detector 113. When positions of the X-ray generator 112 and a reconstruction tomography region are determined, distance information between a voxel to be reconstructed and the X-ray generator 112 may be reflected using the rows 113-1, 113-2, 113-3, 113-4, and 113-5 included in the X-ray detector 113.

According to certain embodiments, the CT system 100 may select a filter kernel based on which row a filter kernel applied to each raw data is acquired. For example, the CT system 100 may select the filter kernels 1210a and 1220a shown in the top left of FIG. 12 as filter kernels to be applied to the raw data acquired using the row 113-3. As another example, the CT system 100 may select the filter kernels 1210c and 1220c shown in the bottom left of FIG. 12 as filter kernels to be applied to the raw data acquired using the row 113-2.

According to certain embodiments, the CT system 100 may select filter kernels to be applied to raw data acquired using a first row based on a position of the first row detecting an X-ray transmitted through a voxel among a plurality of rows included in the X-ray detector 113.

According to certain embodiments, the CT system 100 may generate a filter image by applying a filter kernel selected to correspond to each row to raw data corresponding to each row.

According to certain embodiments, the CT system 100 may generate a tomography image by back-projecting the generated filter image.

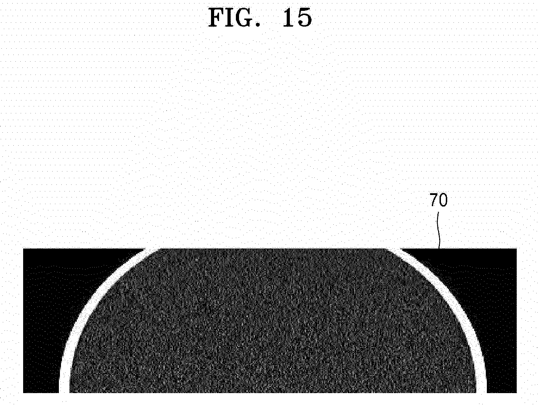

FIG. 4 illustrates an example of the reconstructed tomography image 60 according to the related art. FIG. 15 illustrates an example of a reconstructed tomography image 70, according to an embodiment of the present disclosure.

Referring to FIG. 4, the bright region 61 and the dark region 62 are generated together in the tomography image 60. That is, in the reconstructed tomography image 60 according to the related art, noise included in the tomography image 60 is non-uniformly generated in a zebra pattern.

Referring to FIG. 15, unlike the tomography image 60 shown in FIG. 4, the reconstructed tomography image 70 according to an embodiment of the present disclosure does not have non-uniformly generated noise. That is, according to embodiments of the present disclosure, a uniform image may be acquired in which a bright region and a dark region do not exist separately in the tomography image 70. Thus, a reader may clearly read the tomography image 70.

The disclosed embodiments may be implemented in a software program that includes instructions stored on a computer-readable storage medium.

The computer may include a CT system according to an embodiment as an apparatus capable of calling stored instructions from a storage medium and operating according to the disclosed embodiment according to the called instructions.

The computer-readable storage medium may be provided in the form of a non-transitory storage medium. Here, 'non-temporary' means that the storage medium does not include a signal and is tangible, but does not distinguish data from being stored semi-permanently or temporarily on the storage medium.

Further, the CT system or method according to the disclosed embodiments may be provided in a computer program product. The computer program product may be traded between a seller and a purchaser as a commodity.

Also, the computer program product may include a software program and a computer readable storage medium having stored thereon the software program. For example, the computer program product may include a product (e.g., a downloadable application) in the form of the software program electronically distributed via a manufacturer of the CT system or an electronic marketplace (e.g., Google Play Store and App Store). For electronic distribution, at least part of the software program may be stored on the storage medium or may be created temporarily. In this case, the storage medium may be a server of a manufacturer, a server of an electronic market, or a storage medium of a relay server for temporarily storing the SW program.

Also, the computer program product may include a storage medium of a server or a storage medium of a terminal, in a system consisting of a server and a terminal (e.g., the CT system). Alternatively, when there is a third apparatus (e.g., a smart phone) in communication with the server or the terminal, the computer program product may include a storage medium of the third apparatus. Alternatively, the computer program product may include a software program itself transmitted from the server to the terminal or the third apparatus, or transmitted from the third apparatus to the terminal.

In this case, one of the server, the terminal, and the third apparatus may execute the computer program product to perform the method according to the disclosed embodiments. Alternatively, two or more of the server, the terminal and the third apparatus may execute the computer program product to distribute and perform the method according to the disclosed embodiments.

For example, the server (e.g., a cloud server or an artificial intelligence server, etc.) may execute the computer program product stored on the server to control the terminal communicatively coupled to the server to perform the method according to the disclosed embodiments.

As another example, the third apparatus may execute the computer program product to control a terminal communicatively coupled to the third apparatus to perform the method according to the disclosed embodiment. As a specific example, the third apparatus may control the CT system by remote control to irradiate an X-ray to an object and generate an image of a site inside the object based on information of the X-ray transmitted through the object and detected by an X-ray detector.

As another example, the third apparatus may execute the computer program product to directly perform the method according to the disclosed embodiment based on a value input from an auxiliary apparatus (e.g., a gantry of the CT system). As a specific example, the auxiliary device may irradiate an X-ray to the object and acquire the radiation information detected after being transmitted through the object. The third apparatus may receive the radiation information detected from the auxiliary apparatus, and generate an image of an internal site of the object based on the input radiation information.

When the third apparatus executes the computer program product, the third apparatus may download the computer program product from the server and execute the downloaded computer program product. Alternatively, the third apparatus may execute a computer program product provided in a preloaded manner to perform the method according to the disclosed embodiments.

While embodiments of the present disclosure have been particularly shown and described with reference to the accompanying drawings, it will be understood by those of ordinary skill in the art that various changes in form and details may be made therein without departing from the spirit and scope of the disclosure as defined by the appended claims. The disclosed embodiments should be considered in descriptive sense only and not for purposes of limitation.

Although the present disclosure has been described with various embodiments, various changes and modifications may be suggested to one skilled in the art. It is intended that the present disclosure encompass such changes and modifications as fall within the scope of the appended claims.

## Claims

1. A method of acquiring a tomography image, the method comprising:
acquiring a plurality of raw data by detecting, by an X-ray detector, an X-ray irradiated to an object from an X-ray generator a plurality of times;
setting at least a part of the object as a region of interest; and
reconstructing the tomography image comprising the region of interest, based on the plurality of raw data,
wherein the reconstructing of the tomography image comprises:
selecting filter kernels based on distances between positions of the X-ray generator that irradiated the X-ray to the region of interest and positions of voxels included in the region of interest;
generating filter images by applying the selected filter kernels to the raw data corresponding to the positions of the X-ray generator that irradiated the X-ray; and
reconstructing the tomography image from the filter images by back-projecting the filter images.

2. The method of claim 1, wherein the selecting of the filter kernels comprises:
selecting a first filter kernel corresponding to a first distance based on the first distance between a position of a first voxel included in the region of interest and a first position of the X-ray generator that irradiated the X-ray,
wherein the generating of the filter images comprises: generating a first filter image by applying the selected first filter kernel to first raw data corresponding to the first position, and
wherein the reconstructing of the tomography image comprises reconstructing the first voxel by back-projecting the first filter image.

3. The method of claim 2, wherein the selecting of the first filter kernel comprises selecting the first filter kernel based on at least one of a longest distance and a shortest distance among distances between the positions of the X-ray generator that irradiated the X-ray and the position of the first voxel.

4. The method of claim 3, wherein the selecting of the first filter kernel comprises selecting the first filter kernel by comparing at least one of the longest distance and the shortest distance with a first distance.

5. The method of claim 2, wherein the generating of the filter images comprises:
when there are a plurality of first filter kernels, generating a plurality of first filter images by applying the plurality of first filter kernels to the first raw data; and
generating a second filter image by combining the plurality of generated first filter images.

6. The method of claim 1, wherein the selecting of the filter kernels comprises:
receiving an input from a user to select a set of filter kernels including at least one filter kernel having predetermined noise and sharpness; and
selecting at least one of filter kernels included in the selected set of filter kernels, based on the distances between the positions of the X-ray generator that irradiated the X-ray to the region of interest and the positions of the voxels included in the region of interest.

7. The method of claim 1, wherein the selecting of the filter kernels comprises:
generating a plurality of filter kernels by applying a kernel modulation filter to at least one of the selected filter kernels; and
selecting at least one of the generated plurality of filter kernels, based on the distances between the positions of the X-ray generator that irradiated the X-ray to the region of interest and the positions of the voxels included in the region of interest.

8. A computer program product comprising a non-transitory computer readable storage medium, wherein the non-transitory computer readable storage medium comprises instructions to perform:
acquiring a plurality of raw data by detecting, by an X-ray generator, an X-ray irradiated to an object from an X-ray generator a plurality of times;
setting at least a part of the object as a region of interest; and
reconstructing a tomography image comprising the region of interest, based on the plurality of raw data,
wherein the reconstructing of the tomography image comprises:
selecting filter kernels based on distances between positions of the X-ray generator that irradiated the X-ray to the region of interest and positions of voxels included in the region of interest;
generating filter images by applying the selected filter kernels to the raw data corresponding to the positions of the X-ray generator that irradiated the X-ray; and
reconstructing the tomography image from the filter images by back-projecting the filter images.

9. An apparatus for acquiring a tomography image, the apparatus comprising:
an X-ray generator configured to irradiate an X-ray to an object a plurality of times;
an X-ray detector configured to acquire a plurality of raw data by detecting the irradiated X-ray; and
a processor configured to set at least a part of the object as a region of interest and reconstruct the tomography image comprising the region of interest based on the plurality of raw data,
wherein the processor is configured to select filter kernels based on distances between positions of the X-ray generator that irradiated the X-ray to the region of interest and positions of voxels included in the region of interest, generate filter images by applying the selected filter kernels to the raw data corresponding to the positions of the X-ray generator that irradiated the X-ray, and reconstruct the tomography image from the filter images by back-projecting the filter images.

10. The apparatus of claim 9, wherein the processor is configured to select a first filter kernel corresponding to a first distance based on the first distance between a position of a first voxel included in the region of interest and a first position of the X-ray generator that irradiated the X-ray, generate a first filter image by applying the selected first filter kernel to first raw data corresponding to the first position, and reconstruct the first voxel by back-projecting the first filter image.

11. The apparatus of claim 10, wherein the processor is configured to select the first filter kernel based on at least one of a longest distance and a shortest distance among distances between the positions of the X-ray generator that irradiated the X-ray and the position of the first voxel.

12. The apparatus of claim 11, wherein the processor is configured to select the first filter kernel by comparing at least one of the longest distance and the shortest distance with a first distance.

13. The apparatus of claim 10, wherein the processor is configured to, when there are a plurality of first filter kernels, generate a plurality of first filter images by applying the plurality of first filter kernels to the first raw data; and generate a second filter image by combining the plurality of generated first filter images.

14. The apparatus of claim 9, further comprising a user input interface configured to receive an input from a user to select a set of filter kernels including at least one filter kernel having predetermined noise and sharpness,
wherein the processor is configured to select at least one of filter kernels included in the selected set of filter kernels based on the distances between the positions of the X-ray generator that irradiated the X-ray to the region of interest and the positions of the voxels included in the region of interest.

15. The apparatus of claim 9, wherein the processor is configured to:
generate a plurality of filter kernels by applying a kernel modulation filter to at least one of the selected filter kernels; and
select at least one of the generated plurality of filter kernels based on the distances between the positions of the X-ray generator that irradiated the X-ray to the region of interest and the positions of the voxels included in the region of interest.
